**Europäisches Patentamt**

**European Patent Office**

**Office européen des brevets**

(19)

(11) Veröffentlichungsnummer : **0 013 558**
**B1**

(12) **EUROPÄISCHE PATENTSCHRIFT**

(45) Veröffentlichungstag der Patentschrift :
30.09.81

(51) Int. Cl.³ : **C 07 C143/30**, C 07 C139/00

(21) Anmeldenummer : 80100025.8

(22) Anmeldetag : 04.01.80

(54) Verfahren zur Herstellung von Naphthalin-1,3,5-trisulfonsäure.

(30) Priorität : 13.01.79 DE 2901178

(43) Veröffentlichungstag der Anmeldung :
23.07.80 (Patentblatt 80/15)

(45) Bekanntmachung des Hinweises auf die Patenterteilung : 30.09.81 Patentblatt 81/39

(84) Benannte Vertragsstaaten :
AT BE CH DE FR GB IT NL

(56) Entgegenhaltungen :
EP - A - 0 000 155
FR - A - 2 204 596
FR - A - 2 386 519
FR - A - 2 387 947

(73) Patentinhaber : BAYER AG
Zentralbereich Patente, Marken und Lizenzen
D-5090 Leverkusen 1, Bayerwerk (DE)

(72) Erfinder : Behre, Horst, Dr.
Im Heilsiefen 4
D-5068 Odenthal (DE)
Erfinder : Blank, Heinz Ulrich, Dr.
Am Geusfeld 35
D-5068 Odenthal (DE)
Erfinder : Schenk, Wolfgang, Dr.
Odenthaler Strasse 62/64
D-5090 Leverkusen (DE)

Verfahren zur Herstellung von Naphthalin-1,3,5-trisulfonsäure

Die vorliegende Erfindung betrifft ein Verfahren zur Herstellung von Naphthalin-1,3,5-trisulfonsäure durch Sulfierung eines Produktgemisches, das durch Umsetzung von Naphthalin mit Schwefeltrioxid in einem inerten Lösungsmittel erhalten wurde.

In FIAT Final Report Nr. 1016, Seite 42 bis 44, wird die Herstellung von Naphthalin-1,3,5-trisulfonsäure wie folgt beschrieben :

Naphthalin wird bei 30 bis 35 °C unter gleichzeitigem Einlaufen von 65 %igem Oleum in Monohydrat (= 100 %ige $H_2SO_4$) eingetragen. Die Reaktionsmischung wird eine Stunde bei 50 °C, eine Stunde bei 70 °C und sieben Stunden bei 90 °C gehalten, bis vollständige Lösung eingetreten ist. Die Ausbeute an Naphthalin-1,3,5-trisulfonsäure beträgt bei diesem Verfahren ca. 68 % bezogen auf eingesetztes Naphthalin. Daneben werden ca. 21 % Naphthalin-1,3,6-trisulfonsäure und ca. 4 bis 5 % Naphthalin-1,3,7-trisulfonsäure gebildet (siehe Beispiel 6).

In Ullmann's Enzyklopädie der technischen Chemie, 3. Auflage, 12. Band, Seiten 596 und 631 und in der dort zitierten Literatur wird beschrieben, daß man Naphthalin-1,3,5-trisulfonsäure durch Sulfierung von Naphthalin-1,5-disulfonsäure mit Oleum herstellen kann. Bei diesem Verfahren ist es nachteilig, daß die Naphthalin-1,5-disulfonsäure zuvor als freie Säure in Form ihres Tetrahydrates oder als Dinatriumsalz in Form des Dihydrats isoliert werden muß. Bei der Weitersulfierung zur Naphthalin-1,3,5-trisulfonsäure sind daher erhebliche Mengen Oleum erforderlich, um das Kristallwasser zu binden. Daraus resultieren ungünstige Raum-Zeit-Ausbeuten und ein hoher Verbrauch an Oleum. Wegen der unbefriedigenden Ausbeute an isolierter Naphthalin-1,5-disulfonsäure bzw. deren Dinatriumsalz ist außerdem die Ausbeute an Naphthalin-1,3,5-trisulfonsäure, bezogen auf Naphthalin, ungünstig.

Es wurde nun ein Verfahren zur Herstellung von Naphthalin-1,3,5-trisulfonsäure aus 1,5-disulfiertem Naphthalin gefunden, das dadurch gekennzeichnet ist, daß man 1,5-disulfiertes Naphthalin in Form eines Gemisches, das erhalten worden ist durch Zusammengabe von Naphthalin und $SO_3$ in Gegenwart eines inerten Lösungsmittels bei Temperaturen im Bereich − 40 bis + 20 °C, wobei das Verhältnis von zugegebenem $SO_3$ zu zugegebenem Naphthalin während der gesamten Zugabe im Bereich von 2,5 bis 10 Mol $SO_3$ pro Mol Naphthalin gelegen hat, in wasserfreier Schwefelsäure mit Oleum unter vorheriger oder gleichzeitiger Abtrennung des inerten Lösungsmittels bei 60 bis 110 °C sulfiert.

Das Einsatzprodukt für das erfindungsgemäße Verfahren kann vorzugsweise erhalten werden, indem man Naphthalin und $SO_3$ jeweils getrennt in einem inerten Lösungsmittel, vorzugsweise in einem aliphatischen Chlorkohlenwasserstoff mit 1 bis 3 C-Atomen, insbesondere Dichlormethan, löst und beide Lösungen bei ca. − 10 bis − 5 °C vereinigt, wobei während der gesamten Zugabe ein Molverhältnis von zugegebenem $SO_3$ zu zugegebenem Naphthalin im Bereich von 2,5 bis 3,6, bevorzugt im Bereich von 3,0 bis 3,2, eingehalten wird. Dabei bildet sich ein Sulfierprodukt von Naphthalin, welches sich in fester Form abscheidet und $H_2SO_4$ und gegebenenfalls $SO_3$ enthält. Das Sulfierprodukt fällt als Suspension in dem verwendeten Lösungsmittel an.

Dieses Sulfierprodukt des Naphthalins, im folgenden auch als Armstrongsäure-Anhydrid oder AS-AN bezeichnet, ist ein Gemenge oligomerer Anhydride von Naphthalin-di- und -trisulfonsäuren mit $H_2SO_4$ und gegebenenfalls $SO_3$. Wenn man die im AS-AN vorhandenen sulfierten Naphthalineinheiten formal betrachtet als Naphthalinsulfonsäuren und die $H_2SO_4$ als $SO_3$ auffaßt, so kann das AS-AN beispielsweise 69 bis 88 Gew.-% Naphthalin-di- und -trisulfonsäuren und 31 bis 12 Gew.-% $SO_3$ enthalten und enthält im allgemeinen, bezogen auf die Gesamtmenge an Naphthalinsulfonsäuren, über 65 Gew.-% Naphthalin-1,5-disulfonsäure.

Bevorzugt werden in das erfindungsgemäße Verfahren Gemische eingesetzt, die formal betrachtet, 70 bis 82 Gew.-% Naphthalin-1,5-disulfonsäure, bezogen auf die Gesamtmenge an Naphthalinsulfonsäuren, enthalten. Ein besonders bevorzugtes AS-AN enthält :

75-80 Gew.-% Naphthalin-1,5-disulfonsäure

0- 3 Gew.-% Naphthalin-1,3-disulfonsäure

5-15 Gew.-% Naphthalin-1,6-disulfonsäure

2- 5 Gew.-% Naphthalin-1,7-disulfonsäure

0- 5 Gew.-% Naphthalin-1,3,5-trisulfonsäure

0- 5 Gew.-% Naphthalin-1,3,6-trisulfonsäure

0- 1 Gew.-% Naphthalin-1,3,7-trisulfonsäure

0- 1 Gew.-% Naphthalin-1-sulfonsäure

jeweils bezogen auf die Summe aller Naphthalinderivate und gerechnet als freie Sulfonsäuren.

Darüberhinaus kann das AS-AN in sehr geringen Mengen weitere, nicht näher identifizierte Dinaphthylsulfone und Dinaphthylsulfonsulfonsäuren enthalten.

Das Gemenge oligomerer Anhydride von Naphthalin-di- und -trisulfonsäuren mit $H_2SO_4$ und gegebenenfalls $SO_3$ fällt bei der Herstellung als Suspension im jeweils verwendeten Lösungsmittel an. Um aus dieser Suspension, deren feste Anteile, wie vorstehend ausgeführt, überwiegend 1,5-disulfierte Naphthalineinheiten enthalten, Naphthalin-1,3,5-trisulfonsäure zu erhalten, wird sie in wasserfreier Schwefelsäure und gegebenenfalls Oleum auf Temperaturen im Bereich 60 bis 110 °C gebracht. Wenn dieses Gemisch zwischen 3 und 5 Mol $SO_3$ pro Mol ursprünglich eingesetztes Naphthalin enthält, ist es nicht erforderlich, beispielsweise zusätzliches $SO_3$ oder Wasser oder verdünnte Schwefelsäure einzusetzen.

Das inerte Lösungsmittel kann zu einem beliebigen Zeitpunkt entfernt werden, bevorzugt bevor die Bildung von Naphthalin-1,3,5-trisulfonsäure in nennenswertem Umfang beginnt.

Wenn in dem Reaktionsgemisch, welches das Armstrongsäureanhydrid und wasserfreie Schwefelsäure und gegebenenfalls Oleum enthält, mehr als 5 Mol $SO_3$ pro Mol ursprünglich eingesetztes Naphthalin vorliegen, ist es vorteilhaft, beispielsweise soviel Wasser oder verdünnte Schwefelsäure zusetzen, bis das Molverhältnis von $SO_3$ zu ursprünglich eingesetztem Naphthalin im Bereich 3:1 bis 5:1 liegt. In entsprechender Weise ist es vorteilhaft, beispielsweise $SO_3$ oder Oleum zuzusetzen, wenn in dem vorstehend angegebenen Reaktionsgemisch weniger als 3 Mol $SO_3$ pro Mol ursprünglich eingesetztes Naphthalin vorliegen.

Wenn man in das erfindungsgemäße Verfahren AS-AN einsetzt, das nach der vorstehend als bevorzugt beschriebenen Arbeitsweise erhalten worden ist, ist es im allgemeinen vorteilhaft, wenn man in die Sulfierung zur Bildung von Naphthalin-1,3,5-trisulfonsäure $SO_3$ zufügt. Dabei kann das $SO_3$ beispielsweise als solches oder in Form von Oleum eingesetzt werden. Man Kann auch so verfahren, daß man zur Bildung der Naphthalin-1,3,5-trisulfonsäure das Einsatzgemisch in Oleum einbringt. Die Menge an zugefügtem $SO_3$ wird im allgemeinen in Abhängigkeit vom Gehalt an $SO_3$ des zum Einsatz kommenden AS-AN eingestellt. Beispielsweise kann man pro Mol Naphthalin in Form des 1,5-disulfierten Naphthalins (AS-AN) 0 bis 1,0 Mol $SO_3$, vorzugsweise 0,1 bis 0,8 Mol $SO_3$, z.B. in Form von Oleum zufügen. Hierfür ist beispielsweise Oleum mit einem $SO_3$-Gehalt von 5 bis 100 Gew.-% geeignet, insbesondere das im Handel erhältliche ca. 65 %ige Oleum.

Die Temperatur für die Bildung der Naphthalin-1,3,5-trisulfonsäure wird im Bereich von 60 bis 110 °C eingestellt. Temperaturen im Bereich von 80 bis 100 °C, insbesondere von 85 bis 95 °C sind bevorzugt.

Die Reaktionszeiten zur Bildung der Naphthalin-1,3,5-trisulfonsäure hängen im wesentlichen von der Reaktionstemperatur und der $SO_3$-Konzentration im Reaktionsgemisch ab. Bei relativ hohen Temperaturen und relativ hohen $SO_3$-Konzentrationen kann eine relativ kurze Reaktionszeit, bei relativ niedrigen Temperaturen und relativ niedrigen $SO_3$-Konzentrationen eine längere Reaktionszeit gewählt werden. Vorzugsweise liegt die Reaktionszeit im Bereich zwischen 5 bis 10 Stunden, insbesondere bei 6 bis 8 Stunden.

Die Durchführung der Bildung von Naphthalin-1,3,5-trisulfonsäure kann in verschiedenen Varianten erfolgen. Drei Varianten sind im folgenden detailliert angegeben :

Variante 1

Aus dem Einsatzprodukt wird das inerte Lösungsmittel entfernt bevor man es mit wasserfreier Schwefelsäure und gegebenenfalls $SO_3$ zusammenbringt. Dies kann z.B. durch Filtration mit anschließender Trocknung oder nur durch Trocknung erfolgen. Das so abgetrennte Lösungsmittel kann erneut bei der Sulfierung von Naphthalin zu AS-AN verwendet werden. Das trockene Gemisch (AS-AN) kann dann in vorgelegte wasserfreie Schwefelsäure eingetragen werden. Beispielsweise kann man 1 bis 10 Mol Monohydrat (= 100 %ige $H_2SO_4$), vorzugsweise 2 bis 4 Mol Monohydrat pro Mol Naphthalin in Form von AS-AN vorlegen. Danach kann gegebenenfalls $SO_3$, vorzugsweise in Form von Oleum, eindosiert werden. Das Eintragen von AS-AN und das Eindosieren von Oleum kann simultan, wechselweise in kleinen Portionen oder nacheinander erfolgen. Die Dosierung kann bei Raumtemperatur oder vorzugsweise bei erhöhter Temperatur, erfolgen, beispielsweise bei 30 bis 90 °C. Die Dosierzeiten werden vorzugsweise so gewählt, daß das Reaktionsgemisch gut rührbar und pumpbar bleibt.

Dies ist im allgemeinen bei Dosierzeiten im Bereich von 15 Minuten bis 2 Stunden der Fall. Nach beendeter Dosierung wird die Sulfierung zur Naphthalin-1,3,5-trisulfonsäure vorzugsweise bei 85 bis 95 °C im Verlauf von 5 bis 10 Stunden, vorzugsweise 6 bis 8 Stunden, durchgeführt.

Variante 2

Dem Einsatzprodukt wird zunächst wasserfreie Schwefelsäure und ein Teil ausreagiertes Sulfiergemisch aus einem vorhergehenden Ansatz nacheinander, simultan oder nach vorheriger Vermischung zugesetzt. Dabei bilden sich zwei flüssige Phasen aus, eine Säurephase und eine Lösungsmittelphase. Man kann beispielsweise wasserfreie Schwefelsäure in einer solchen Menge zufügen, wie sie bei Variante 1 angegeben ist und zusätzlich etwa 10 bis 70 Gew.-% des rohen ausreagierten Sulfiergemisches aus einem vorhergehenden Ansatz. Es ist vorteilhaft, wenn die sich bildende Säurephase etwa 50 bis 250 Gew.-% der Lösungsmittelphase entspricht. Die Hauptmenge des inerten Lösungsmittels, beispielsweise etwa 80 bis 95 %, werden dann durch Dekantieren abgetrennt. Der Dekantationsrückstand wird dann durch Destillation von restlichem inertem Lösungsmittel befreit, beispielsweise durch Destillation im Vakuum. Die Dekantierung erfolgt vorzugsweise bei ca. 15 bis 25 °C, das Abdestillieren des restlichen Lösungsmittels vorzugsweise bei 10 bis 30 °C und 13 bis 133 mbar. Das so abgetrennte Lösungsmittel kann erneut bei der Sulfierung von Naphthalin zu AS-AN verwendet werden. Anschließend wird durch Einstellen von Temperaturen im Bereich von 60 bis 110 °C und gegebenenfalls nach Zugabe von Oleum die Bildung von Naphthalin-1,3,5-trisulfonsäure im Verlaufe von 5 bis 10 Stunden bewerkstelligt. Nach beendeter Sulfierung werden etwa zwischen 10 und 70 Gew.-% des rohen, ausreagierten Sulfiergemisches in die Phasentrennung zurückgeführt.

Variante 3

Man verfährt wie bei Variante 1 oder 2, trennt das inerte Lösungsmittel jedoch erst dann ab, beispielsweise durch Destillation, wenn man die Temperatur innerhalb des Bereiches von 60 bis 110 °C einstellt.

Besonders bevorzugt wird gemäß der Variante 1 oder der Variante 2 gearbeitet. Man kann diese Varianten auch ohne Schwierigkeiten kontinuierlich durchführen.

Gemäß dem erfindungsgemäßen Verfahren kann Naphthalin-1,3,5-trisulfonsäure in Ausbeuten von ca. 83 %, bezogen auf Naphthalin, d.h. in wesentlich höheren Ausbeuten als gemäß dem aufgezeigten Stand der Technik, gewonnen werden. Das nach dem erfindungsgemäßen Verfahren vorliegende ausreagierte Sulfiergemisch enthält neben Naphthalin-1,3,5-trisulfonsäure ca. 15 % Naphthalin-1,3,6-trisulfonsäure und geringe Mengen Naphthalin-1,3,7-trisulfonsäure und Naphthalin-1,3,5,7-tetrasulfonsäure (jeweils bezogen auf eingesetztes Naphthalin). Gegenüber den bisher bekannten Verfahren werden beim erfindungsgemäßen Verfahren hohe Raum-Zeit-Ausbeuten erreicht und der Verbrauch an Hilfsstoffen, z.B. Monohydrat, ist wesentlich verringert.

Das nach Durchführung des erfindungsgemäßen Verfahrens vorliegende Produkt kann ohne Isolierung der Naphthalin-1,3,5-trisulfonsäure auf beliebige Weise weiterverwendet werden. Beispielsweise ist es zur Herstellung der wichtigen Farbstoffzwischenprodukte Naphthamintrisäure K (Melansäure) und K-Säure geeignet, wobei die Schritte Nitrierung, Kreidung, Reduktion und Abscheidung von Naphthamintrisäure K und Überführung der Naphthamintrisäure K in K-Säure entsprechend FIAT-Final-Report Nr. 1016, Seite 42 bis 44 oder auf andere Weise durchgeführt werden können.

Beispiele

Beispiel 1

In einem 2-1-Vierhals-Kolben, ausgestattet mit einer Dosierschnecke, einem Dosiertropftrichter, Innenthermometer und Säbelrührer, werden 350 g 100 Gew.-%ige $H_2SO_4$ vorgelegt. Über die Dosierschnecke werden im Verlauf von ca. 30 Minuten unter Rühren 369 g des Produktes (AS-AN) zugegeben, das wie folgt erhalten wurde:

128 g Naphthalin (1,0 Mol) wurden in 640 g Dichlormethan gelöst, gleichzeitig 243 g flüssiges $SO_3$ in 950 g Dichlormethan. Beide Lösungen wurden simultan in 100 g vorgelegtes Dichlormethan so einlaufen gelassen, daß die Temperatur zwischen −5 und −10 °C gehalten werden konnte. Nach beendeter Zusammengabe wurde die Reaktionsmischung 1,5 Stunden bei −5 bis −10 °C gehalten und anschließend im Vakuum zur Trockene eingedampft. Das so erhaltene Produkt hatte formal betrachtet, folgende Zusammensetzung:

59,6 Gew.-% Naphthalin-1,5-disulfonsäure

0,2 Gew.-% Naphthalin-1,3-disulfonsäure

5,2 Gew.-% Naphthalin-1,6-disulfonsäure

2,2 Gew.-% Naphthalin-1,7-disulfonsäure

2,5 Gew.-% Naphthalin-1,3,5-trisulfonsäure

5,8 Gew.-% Naphthalin-1,3,6-trisulfonsäure

0,5 Gew.-% Naphthalin-1,3,7-trisulfonsäure

24,0 Gew.-% $SO_3$.

Während der Zudosierung des AS-AN wurde die Reaktionsmischung auf 70 °C erwärmt. Anschließend wurden über den Dosiertropftrichter 42 g Oleum 65 %, eingetropft und die Reaktionsmischung 7 Stunden bei 90 °C gerührt.

In der nach der Sulfierreaktion vorliegenden Reaktionsmischung wurden durch Hochdruckflüssigkeitschromatographie folgende Gehalte bestimmt:

Naphthalin-1,3,5-trisulfonsäure : 40,2 Gew.-%

Naphthalin-1,3,6-trisulfonsäure : 7,4 Gew.-%

Naphthalin-1,3,7-trisulfonsäure : 1,4 Gew.-%

Naphthalin-1,3,5,7-tetrasulfonsäure :
0,2 Gew.-%

Schwefelsäure : ca. 51 Gew.-%.

Die Ausbeute an Naphthalin-1,3,5-trisulfonsäure betrug 82 % bezogen auf Naphthalin.

Das resultierende Naphthalin-trisulfonsäure-Isomeren-Gemisch wurde ohne Zwischenisolierung der Naphthalin-1,3,5-trisulfonsäure durch Nitrierung, Kreidung und Reduktion gemäß FIAT-Final Report Nr. 1016, Seiten 42 bis 44 zur Naphthamintrisäure K weiterverarbeitet.

Beispiel 2

Eine wie in Beispiel 1 durchgeführte Reaktion, bei der ein Sulfierungsprodukt eingesetzt wurde, das wie in Beispiel 1 erhalten wurde, bei dem jedoch 264 g $SO_3$ in 1 025 g Dichlormethan gelöst wurde, und das formal betrachtet, folgende Zusammensetzung aufwies:

56,0 Gew.-% Naphthalin-1,5-disulfonsäure

9,1 Gew.-% Naphthalin-1,3,5-trisulfonsäure

16,2 Gew.-% weitere Naphthalin-disulfonsäuren und Naphthalin-trisulfonsäuren

19,0 Gew.-% $SO_3$
und bei dem die Reaktionsmischung nach der Zugabe von 42 g Oleum 65 % 10 Stunden bei 85 °C gerührt wurde, ergab eine Ausbeute an Naphthalin-1,3,5-trisulfonsäure von 83 %, bezogen auf Naphthalin.

Beispiel 3

Es wurde wie in Beispiel 1 verfahren, jedoch wurde die Reaktionsmischung nach Zugabe von 42 g Oleum 65 % 12 Stunden bei 80 °C gerührt. Die Ausbeute an Naphthalin-1,3,5-trisulfonsäure betrug 80 %, bezogen auf Naphthalin.

Beispiel 4

In einem 3-1-Dreihalskolben, ausgestattet mit einem Tropftrichter, Innenthermometer und Säbelrührer wurde eine Suspension von 369 g AS-AN in 900 g Dichlormethan mit der in Beispiel 1 angegebenen Zusammensetzung vorgelegt.

In einem separaten Kolben wurden 194 g rohes Sulfierreaktionsgemisch (Rückführung aus vorherigem Sulfieransatz) und 350 g $H_2SO_4$ 100 %

vorgelegt und vermischt. Zur Suspension aus AS-AN und Dichlormethan wurde nun bei 20 °C das Gemisch aus dem separaten Kolben zugegeben und langsam gerührt, bis sich das Dichlormethan in der oberen Schicht klar abtrennt, wonach es abgesaugt (abdekantiert) wurde. Das dadurch erhaltene Dichlormethan, ca. 725 g, wurde ohne Aufarbeitung in die Sulfonierung von Naphthalin recyclisiert. Die restlichen 175 g Dichlormethan wurden bei 10 bis 30 °C und 27 mbar abdestilliert.

Die Trisulfierung erfolgt durch Zugabe von 42 g Oleum 65 % zum Destillationsrückstandsbrei und 4-stündigem Erwärmen der Reaktionsmischung auf 95 °C. Die Ausbeute an Naphthalin-1,3,5-trisulfonsäure betrug 82 %, bezogen auf Naphthalin. Der Gehalt an organisch-gebundenem Chlor im Sulfiergemisch war geringer als 0,2 %.

Etwa ein Viertel der erhaltenen Reaktionsmischung wurde in die Zweiphasentrennung zurückgeführt, die restlichen drei Viertel wurden nach FIAT-Final Report 1016, Seiten 42 bis 44, zur Naphthamintrisäure K weiterverarbeitet.

**Beispiel 5**

Es wurde wie in Beispiel 4 verfahren, jedoch wurden in dem separaten Kolben 388 g rohes Sulfonierungsreaktionsgemisch und 350 g $H_2SO_4$ 100 % vorgelegt und vermischt. Für die Trisulfierung wurde die Reaktionsmischung 4 Stunden bei 100 °C gerührt.

Es wurden 85 % des eingesetzten Dichlormethans durch Dekantieren und 15 % durch Destillation zurückgewonnen. Die Ausbeute an Naphthalin-1,3,5-trisulfonsäure betrug 81 %, bezogen auf Naphthalin.

**Beispiel 6 (entsprechend FIAT-Final Report Nr. 1016, Seiten 42 bis 44)**

In einer wie in Beispiel 1 beschriebenen Apparatur wurden 225 g 100 %ige $H_2SO_4$ vorgelegt. Im Verlaufe von ca. 1 Stunde wurde bei 30 bis 35 °C 128 g Naphthalin unter gleichzeitigem Zulaufen von 410 g Oleum 65 % eindosiert. Die Reaktionsmischung wurde auf 50 °C geheizt, eine Stunde bei 50 °C, eine Stunde bei 70 °C und sieben Stunden bei 90 °C gehalten.

In der nach der Sulfierreaktion vorliegenden Reaktionsmischung wurden durch Hochdruckflüssigkeitschromatographie folgende Gehalte bestimmt :

Naphthalin-1,3,5-trisulfonsäure : 33,0 Gew.-%
Naphthalin-1,3,6-trisulfonsäure : 10,3 Gew.-%
Naphthalin-1,3,7-trisulfonsäure : 2,1 Gew.-%
Naphthalin-1,3,5,7-tetrasulfonsäure : 0,3 Gew.-%
Schwefelsäure : ca. 54 Gew.-%.

Die Ausbeute an Naphthalin-1,3,5-trisulfonsäure betrug 68 %, bezogen auf Naphthalin.

**Ansprüche**

1. Verfahren zur Herstellung von Naphthalin-1,3,5-trisulfonsäure aus 1,5-disulfiertem Naphthalin, dadurch gekennzeichnet, daß man 1,5-disulfiertes Naphthalin in Form eines Gemisches, das erhalten worden ist durch Zusammengabe von Naphthalin und $SO_3$ in Gegenwart eines inerten Lösungsmittels bei Temperaturen im Bereich − 40 bis + 20 °C, wobei das Verhältnis von zugegebenem $SO_3$ zu zugegebenem Naphthalin während der gesamten Zugabe im Bereich von 2,5 bis 10 Mol $SO_3$ pro Mol Naphthalin gelegen hat, in wasserfreier Schwefelsäure mit Oleum unter vorheriger oder gleichzeitiger Abtrennung des inerten Lösungsmittels bei 60 bis 110 °C sulfiert.

2. Verfahren nach Anspruch 1, dadurch gekennzeichnet, daß man in dem Reaktionsgemisch aus 1,5-disulfiertes Naphthalin enthaltendem Gemisch, wasserfreier Schwefelsäure und Oleum durch Zugabe von Wasser, verdünnter Schwefelsäure oder Oleum ein Molverhältnis von $SO_3$ zu ursprünglich eingesetztem Naphthalin von 3:1 bis 5:1 einstellt.

3. Verfahren nach Anspruch 1 und 2, dadurch gekennzeichnet, daß man von 1,5-disulfierten Naphthalin ausgeht, das erhalten worden ist, indem man Naphthalin und $SO_3$ jeweils getrennt in einem inerten Lösungsmittel löst, beide Lösungen bei − 10 bis − 5 °C vereinigt und während der gesamten Zugabe ein Molverhaltnis von zugegebenem $SO_3$ zu zugegebenem Naphthalin im Bereich von 2,5 bis 3,6 einhält.

4. Verfahren nach Anspruch 1 bis 2, dadurch gekennzeichnet, daß man in die Sulfierung zur Bildung von Naphthalin-1,3,5-trisulfonsäure kein zusätzliches $SO_3$ einsetzt.

5. Verfahren nach Anspruch 3, dadurch gekennzeichnet, daß man in die Sulfierung zur Bildung von Naphthalin-1,3,5-trisulfonsäure 0,1 bis 0,8 Mol $SO_3$ pro Mol Naphthalin in Form des 1,5-disulfierten Naphthalins zufügt.

6. Verfahren nach Anspruch 5, dadurch gekennzeichnet, daß man $SO_3$ in Form von Oleum zufügt.

7. Verfahren nach Anspruch 1 bis 6, dadurch gekennzeichnet, daß man für die Bildung von Naphthalin-1,3,5-trisulfonsäure eine Temperatur im Bereich 80 bis 100 °C einstellt.

8. Verfahren nach Anspruch 1 bis 7, dadurch gekennzeichnet, daß man aus dem Einsatzprodukt das inerte Lösungsmittel entfernt, bevor man es mit wasserfreier Schwefelsäure und gegebenenfalls $SO_3$ zusammenbringt.

9. Verfahren nach Anspruch 1 bis 7, dadurch gekennzeichnet, daß man dem Einsatzprodukt zunächst wasserfreie Schwefelsäure und einen Teil ausreagiertes Sulfie misch zusetzt, dann die Hauptmenge des inerten Lösungsmittels durch Dekantieren und dann das restliche inerte Lösungsmittel durch Destillation abtrennt.

**Claims**

1. Process for the preparation of naphthalene-1,3,5-trisulphonic acid from 1,5-disulphonated naphthalene, characterised in that 1,5-disulphonated naphthalene, in the form of a mixture which has been obtained by mixing naphthalene and $SO_3$ in the presence of an inert solvent at temperatures in the range of $-40$ to $+20\,°C$, the ratio of $SO_3$ added to naphthalene added having been in the range of 2.5 to 10 mols of $SO_3$ per mol of naphthalene during the entire addition, is sulphonated at 60 to 110 °C in anhydrous sulphuric acid with oleum, the inert solvent being separated off beforehand or at the same time.

2. Process according to Claim 1, characterised in that in the reaction mixture consisting of the mixture containing 1.5-disulphonated naphthalene and of anhydrous sulphuric acid and oleum, the molar ratio of $SO_3$ to naphthalene originally employed is adjusted to 3:1 to 5:1 by adding water, dilute sulphuric acid or oleum.

3. Process according to Claim 1 and 2, characterised in that the starting material used is 1,5-disulphonated naphthalene which has been obtained by dissolving naphthalene and $SO_3$, each separately, in an inert solvent, combining the two solutions at $-10$ to $-5\,°C$ and maintaining a molar ratio of $SO_3$ added to naphthalene added in the range of 2.5 to 3.6 during the entire addition.

4. Process according to Claim 1 to 2, characterised in that no additional $SO_3$ is employed in the sulphonation reaction for the formation of naphthalene-1,3,5-trisulphonic acid.

5. Process according to Claim 3, characterised in that 0.1 to 0.8 mol of $SO_3$ per mol of naphthalene in the form of the 1,5-disulphonated naphthalene is added in the sulphonation reaction for the formation of naphthalene-1,3,5-trisulphonic acid.

6. Process according to Claim 5, characterised in that $SO_3$ is added in the form of oleum.

7. Process according to Claim 1 to 6, characterised in that, for the formation of naphthalene-1,3,5-trisulphonic acid, the temperature is adjusted to a temperature in the range of 80 to 100 °C.

8. Process according to Claim 1 to 7, characterised in that the inert solvent is removed from the feed product before it is mixed with anhydrous sulphuric acid and optionnally $SO_3$.

9. Process according to Claim 1 to 7, characterised in that anhydrous sulphuric acid and a portion of completely reacted sulphonation mixture are first added to the feed product, the bulk of the inert solvent is then separated off by decanting and the residual inert solvent is then separated off by distillation.

## Revendications

1. Procédé pour la préparation d'acide naphtalène-1,3,5-trisulfonique à partir de naphtalène 1,5-disulfoné, caractérisé en ce que l'on sulfone dans l'acide sulfurique anhydre, au moyen d'oléum à 60-110 °C, avec séparation préalable ou simultanée du solvant inerte le naphtalène 1,5-disulfoné sous forme d'un mélange qui a été obtenu par mise en présence de naphtalène et de $SO_3$ en présence d'un solvant inerte à des températures de $-40$ à $+20\,°C$, le rapport du $SO_3$ ajouté au naphtalène ajouté ayant été ajusté pendant toute l'addition dans l'intervalle de 2,5 à 10 moles de $SO_3$ par mole de naphtalène.

2. Procédé selon la revendication 1, caractérisé en ce que l'on ajuste un rapport molaire de $SO_3$ au naphtalène utilisé initialement de 3:1 à 5:1 dans le mélange de réaction constitué par le mélange contenant le naphtalène 1,5-disulfoné, l'acide sulfurique anhydre et l'oléum, par addition d'eau, d'acide sulfurique dilué ou d'oléum.

3. Procédé selon les revendications 1 et 2, caractérisé en ce que l'on part de naphtalène 1,5-disulfoné qui a été obtenu en dissolvant séparément le naphtalène et le $SO_3$ dans un solvant inerte, en combinant les deux solutions à environ $-10$ à $-5\,°C$ et en maintenant pendant toute l'addition un rapport molaire du $SO_3$ ajouté au naphtalène ajouté compris dans l'intervalle de 2,5 à 3,6.

4. Procédé selon les revendications 1 et 2, caractérisé en ce que l'on n'utilise pas de $SO_3$ supplémentaire dans la sulfonation pour la formation de l'acide naphtalène-1,3,5-trisulfonique.

5. Procédé selon la revendication 3, caractérisé en ce que l'on ajoute 0,1 à 0,8 mole de $SO_3$ par mole de naphtalène, sous forme du naphtalène 1,5-disulfoné dans la sulfonation pour la formation de l'acide naphtalène-1,3,5-trisulfonique.

6. Procédé selon la revendication 5, caractérisé en ce que l'on ajoute le $SO_3$ sous forme d'oléum.

7. Procédé selon les revendications 1 à 6, caractérisé en ce que l'on ajuste une température de 80 à 100 °C pour la formation de l'acide naphtalène-1,3,5-trisulfonique.

8. Procédé selon les revendications 1 à 7, caractérisé en ce que l'on élimine le solvant inerte du produit utilisé avant de le mettre en contact avec l'acide sulfurique anhydre et éventuellement le $SO_3$.

9. Procédé selon les revendications 1 à 7, caractérisé en ce que l'on ajoute d'abord au produit chargé de l'acide sulfurique et une partie du mélange de sulfonation ayant cessé de réagir, puis on sépare la majeure partie du solvant inerte par décantation et, ensuite, le solvant inerte résiduel par distillation.